# EUROPEAN PATENT APPLICATION

(11) **EP 4 494 760 A1**
(43) Date of publication of application: **22.01.2025**
(21) Application number: 23186026.3
(22) Date of filing: 18.07.2023
(51) Int. Cl.: B01J 29/40, B01J 23/10, B01J 29/90, B01J 38/12, C01B 39/02, C10G 3/00, B01J 37/04, C07C 1/20, C07C 11/02, B01J 23/92, B01J 29/70

(54) **PROCESS AND CATALYST FOR CONVERTING METHANOL INTO HYDROCARBONS**

(71) Applicant: ETH Zurich, 8092 Zurich (CH)
(72) Inventor: VAN BOKHOVEN, Jeroen, 8053 Zürich (CH); PAUNOVIC, Vladimir, 8049 Zürich (CH); PARE, Charles, 55299 Nackenheim (DE)

(57) **Abstract**

The present invention relates to an improved porous solid catalyst (C) adapted for the conversion of methanol and derivatives thereof into low-molecular weight hydrocarbons, based on a zeolite, such as ZSM-5, and on inorganic oxide, such as CeO2, in a preferable weight ratio of zeolite:inorganic oxide between 1:1 and 1:5. It further relates to a conversion process and to a system using such a catalyst.

## Description

### Technical domain

The present invention concerns a catalyst adapted for converting methanol and methanol derivatives into hydrocarbons. In particular it concerns an improved zeolite catalyst adapted for methanol-to-hydrocarbons (MTH) conversion. The present invention further concerns an improved process for converting methanol and methanol derivatives into hydrocarbons.

### Related art

Zeolites, which are microporous crystalline aluminosilicates, are used as catalysts in the conversion of methanol to different products such as gasoline (MTG) and olefines (MTO). In particular, ZSM-5 zeolite have demonstrated good results in converting methanol into hydrocarbons (MTH). It has been for example described in the patent US 5367100. Methanol can be obtained from different feedstocks including biomass, biochar, natural gas, and coal. In the current efforts in valorising the biomass, conversion of methanol represents a promising pathway. Importantly, methanol can be produced from carbon dioxide (CO₂) and renewable hydrogen, which is an attractive route to attain the circular carbon economy and to reduce the CO₂ emissions. However, the current MTH heterogeneous catalysis suffers from an inevitable activity loss during operation, which is primarily caused by coke formation. The catalysts need to be regenerated under harsh conditions, which can be costly. The necessary high temperature above 823 K leads to degradation of the active acid sites and microporous structure of the catalyst. Furthermore, part of the methanol feed is trapped in the coke, which significantly degrades the product yield. Some non-zeolite catalyst components can be used to enhance the lifetime of the zeolite catalyst. To this end, impregnation or ion exchanges approaches can be used. However, incorporation of ions in the zeolite usually occludes the active micropore volume and thus may be detrimental to the activity of the catalyst.

There is thus room for improving the properties of the catalysts used in the methanol-to-hydrocarbons conversion and related processes.

### Short disclosure of the invention

An aim of the present invention is the provision of a catalyst and a process that overcomes the shortcomings and limitations of the state of the art.

It is in particular an aim of the present invention to provide a catalyst, or a catalytic composition adapted for the conversion of methanol and methanol derivatives into hydrocarbons, having an improved lifetime under the conditions of the conversion process. It is in particular an aim of the present invention to provide a catalyst or a catalytic composition having a longer lifetime at lower pressures such as pressure lower than 30 or 10 or 5 or 2 bar. The lifetime of the present catalyst or catalytic composition is for example longer than 150 or 200 or 250 or 300 hours, corresponding to more than 10 or 50, 500, or 20000 grams of methanol converted per gram of catalyst under typical reaction conditions before a regeneration process is needed.

It is a further aim of the present invention to provide a catalyst or a catalytic composition adapted for an improved selectivity of hydrocarbon production. It is for example an objective to maintain or improve the selectivity for hydrocarbons such as alkanes, alkenes, alkynes having less than 15 carbon atoms, preferably less than 10 or 8 or 5 carbon atoms, more preferably of 2 to 4 carbon atoms. An improved selectivity here refers to more than 70%, or more than 80% or more than 85% or more than 90% or 95% of lower-molecular weight hydrocarbons. One of the objectives is to provide a catalyst or a catalytic composition adapted to maintain or promote a low ratio of higher molecular weight hydrocarbons such as polycyclic arenes and related compounds, under the conditions of the conversion process. The ratio of such high-weight molecules is here preferably lower than 10%, or lower than 5%.

It is a further aim of the present invention to provide a catalyst or a catalytic composition having an increased turn-over or an improved throughput capacity such as higher than 30, 50, 500, 700, 900, 1000, 1500, or 20000 grams of methanol per gram of catalyst or catalytic composition.

It is a further aim of the present invention to provide a catalyst or a catalytic composition having an improved stability under the conditions of the conversion and/or under the conditions of the regeneration.

It is a further aim of the present invention to provide a process for conversion of methanol and methanol derivatives into hydrocarbons, in particular into low-molecular weight hydrocarbons. It is a further aim to provide an improved and less costly process for conversion of methanol and methanol derivatives into such hydrocarbons.

According to the invention, these aims are attained by the object of the independent claims, and further defined by the claims depending thereof.

With respect to what is known in the art, the invention provides the advantage of a more selective and/or more sustainable catalysis for the conversion of methanol and related products.

### Short description of the drawings

Exemplar embodiments of the invention are disclosed in the description and illustrated by the following drawings:
- Figure 1a: Diagram showing lifetime enhancement of mixed oxide zeolite (MOZ) catalysts according to an embodiment of the present invention, compared to state of the art.
- Figure 1b: Diagram showing lifetime extension under hydrogen co-feed of unstable zeolites mixed with inorganic oxides according to an embodiment of the present invention, compared with state of the art.
- Figure 2: Diagram showing the regeneration of a catalyst according to an embodiment of the present invention compared to state of the art.
- Figure 3a: Diagram showing improvement of zeolite throughput capacity with CeO₂.
- Figure 3b: Diagram showing selectivity profile for low-molecular weight hydrocarbons, for a catalyst according to an embodiment of the present invention, compared to state of the art.
- Figure 4a: Diagram showing lifetime enhancement of bulk mixed oxides zeolite according to an embodiment of the present invention.
- Figure 4b: Diagram showing lifetime enhancement of zeolite combined with supported inorganic oxides according to an embodiment of the present invention.
- Figure 5: Comparative lifetime enhancement for bulk mixed oxides zeolite and zeolite combined with supported inorganic oxides according to the present disclosure.
- Figure 6: Schematic representation of a reactor adapted for methanol conversion according to the present disclosure.

### Examples of embodiments of the present invention

According to an aspect, the present disclosure relates to an acidic zeolite catalyst, or catalytic composition, used in the conversion of methanol or methanol derivatives into hydrocarbons.

The term methanol derivative denotes compounds having close properties compared to methanol. The methanol derivatives relate to compounds having one carbon atom and one heteroatom selected from oxygen, sulphur, nitrogen, and halogen such as fluorine, chlorine, iodine, and bromine. Methanol derivatives include, CH₃Cl, CH₃Br, CH₃F, CH₃I, CH₃NH₂, CH₃SH, CHzO, CH₂S, CHCl₂, CHCl₃, CCl₄, and related compounds. The term methanol derivatives also includes compounds having one carbon atom and functional group comprising an heteroatom and one additional carbon atom such as dimethyl ether (CH₃OCH₃), and dimethylamine ((CH₃)₂)NH).

Zeolite here denotes microporous crystalline materials, based on silicon, aluminium and oxygen and comprising variable amount of proton H⁺ involved in the catalytic reactions. Zeolites are characterized by their composition as well as their porosity. They are also used as molecular sieves. Examples of zeolites includes the ZSM zeolites such as ZSM-1, ZSM-4, ZSM-5, ZSM-11, ZSM-12, ZSM-23, ZSM-30, ZSM-34, ZSM-48, ZSM-50, ZSM-57, ZSM-58, then Sigma-1 and ITQ-3 zeolites, the SAPO molecular sieves such as SAPO-5, SAPO-8, SAPO-11, SAPO-14, SAPO-16, SAPO-17, SAPO-18, SAPO-20, SAPO-31, SAPO-35, SAPO-36, SAPO-37, SAPO-40, SAPO-41, SAPO-42, SAPO-44, SAPO-47, SAPO-57, SAPO-56, SAPO-59, STA-6, the AIPO molecular sieves such as AIPO-5, AIPO-11, AIPO-18, AIPO-18, AIPO-31, AIPO-34, AIPO-36, AIPO-37, AIPO-46. And less stable zeolites such as ITQ-13, ITQ-33, SUZ-4, MCM-22, EU-1, UZM-9, UZM-12, SAT-7, ZK-5, RHO, RUB-13, UZM-5, SSZ-13, SSZ-16, SSZ-24, SSZ-39, SSZ-52, SSZ-98, BETA, ZSM-35, FAU, ZSM-22, BEA, USY and MOR.

According to an embodiment, the catalyst or the catalytic composition of the present invention comprises ZSM-5 or ZSM-11 or a combination thereof, more preferably ZSM-5 alone. ZSM-5 includes ZSM-5(25), having a Si/AI ratio of 25 and ZSM-5(40), having a Si/AI ratio of 40. Other Si/AI ratios can be envisaged.

According to another embodiment, the catalyst or the catalytic composition of the present disclosure comprises SSZ-13, BETA, FAU, ZSM-22, ZSM-35, BEA, USY and MOR or a combination thereof as a zeolite.

The catalyst here denotes a pure zeolite or a mixture of zeolites, combined with at least one inorganic oxide such as a metal oxide, thus also defining a catalytic composition. Preferably, the present catalyst is homogenously prepared, meaning that its structure, including its porosity, and its composition, are identical in its whole volume. The catalyst is preferably prepared as beads, powder, or slurry, which can be free or supported on a solid support. The catalyst can be presented as millimetre-sized beads or extrudates, as micrometric beads or as a powder having a particle size lower than one micrometre. The present catalyst is thus preferably a microporous solid catalyst.

The inorganic oxides are derived from mono-, di-, tri-, and tetra-oxidation of one of the alkali metals, the alkaline rare earth metals, rare earth metals and transition metals. The inorganic oxides denote for example mono-, or di-, tri- or tetraoxides of lanthanides such as lanthanum, cerium, praseodymium, ytterbium. The inorganic oxides include Sm₂O₃, Y₂O₃, La₂O₃, MgO, CaO, ZrO₂, CeO₂, Ce₂O₃, Mo₂O₃, SrO, BaO, Eu₂O₃, Pr₂O₃, Nd₂O₃, Pm₂O₃, Tb₂O₃, Dy₂O₃,Ho₂O₃, Er₂O₃, SnO₂, ZnO, Mo₂O₃, SiO₂, Al₂O₃, TiO₂, or a mixture thereof, or their doped forms, wherein dopant is one or more alkaline, alkaline earth, transition, or rare-earth metals.

It has been found that the combination of one or several zeolites with at least one inorganic oxide, according to the present disclosure, allows to improve the lifetime of the catalyst or the catalytic composition under same or comparable conditions. Such combination improves for example the lifetime by more than 2, 3, 5, 10 or 20 times compared with the corresponding zeolite without inorganic oxides. Depending on the nature of the inorganic oxide and /or its ratio with the zeolite, the lifetime can be improved by 3 to 56 times, like from 10 to 56, compared with the zeolite alone.

It has been found that the combination of one or several zeolites with at least one inorganic oxide, according to the present disclosure, allows to reduce the formation of aromatic compounds, compared with the use of the zeolite alone, under same or comparable conditions. At the same time, the selectivity for low-molecular weight compounds remains identical or similar to, or improved with respect to the corresponding value obtained with the zeolite alone.

It has been further found that the combination of one or more zeolites with at least one inorganic oxide, as here described, enhances the rate of the coke oxidation under regeneration conditions. The regeneration conditions usually require temperature higher than around 820 K for zeolites without inorganic oxides. According to the present invention, the regeneration of the catalyst or the catalytic composition can occur at temperatures lower than 820 K, or lower than 600 K or lower than 400 K. In a particular embodiment, the temperature of catalyst regeneration can be comprised between 550 and 770 K. The energy spent for the regeneration is thus reduced. Furthermore, the catalyst or catalytic composition is better preserved during the regeneration process and can afford more regeneration cycles. This further allows the use of less common zeolites which would otherwise not be stable enough under the conversion conditions.

It has been also found that the stability of the less stable zeolites, in particular zeolites like SSZ-13, BETA, ZSM-35, FAU, ZSM-22, BEA, USY and MOR, can be improved when combined with at least one inorganic oxide, according to the present disclosure. It results that the lifetime of such zeolites is improved by at least 4 to 56 times compared with the zeolite free of inorganic oxides. This opens more flexibility in the conversion of methanol and methanol derivatives due the larger variety of usable zeolites. For example, by making one of these less-stable zeolites available, an improved selectivity can be obtained for a category of compounds, such as alkenes having from 3 to 5 carbon atoms.

According to an embodiment, a zeolite is mixed with one or several inorganic oxides so as to provide a homogenous preparation. A suitable amount of zeolite, comprising either one or several zeolites, is mixed with a suitable amount of one or several inorganic oxides, and treated so as to provide a homogenous solid catalyst. The treatment includes mechanical treatment such as crushing, pressing and sieving the combined materials. Alternatively or in addition, the treatment can comprise thermal operations such as heating at a suitable temperature. Alternatively or in addition, the treatment can comprise chemical treatment such as washing in water, or other solvent such as polar or non-polar solvents. The treatment may further comprise crystallisation steps where necessary. Preferably, the catalyst or catalytic composition does not comprise organic additives or organometallic additives. Preferably, the catalyst or catalytic composition is free of phosphorous, phosphorous oxides and phosphorous derivatives. The resulting catalyst or catalytic composition is a mixed oxide zeolite (MOZ) wherein the zeolite and the inorganic oxides are mixed in bulk.

Alternatively, one or several inorganic oxides can be supported by the zeolite of the catalyst or one of the zeolite in case several zeolites are present, or by another carrier like a secondary inorganic oxide.

According to an embodiment, one zeolite is combined with only one inorganic oxide.

According to another embodiment, one zeolite is combined with several inorganic oxides, comprising a main inorganic oxide and a secondary inorganic oxide. A main inorganic oxide can represent 0.1% to 90% by weight of the inorganic oxide of the catalyst or the catalytic composition. Alternatively, the main inorganic oxide represents less than 30% or 20% or 10% by weight of the inorganic oxide.

The main inorganic oxide is understood as providing at least one of the above-mentioned advantages, in particular lifetime improvement, turn-over or throughput capacity improvement, stability improvement, selectivity improvement, regeneration improvement. Preferably the main inorganic oxide is selected to provide a combination of some or all the above-described advantages.

The secondary inorganic oxide is understood as being less advantageous than the main inorganic oxide, when taken instead of the main inorganic oxide, with regard to some or all of the above-mentioned benefits. The secondary inorganic oxide can be selected based on its cost, which is preferably lowerthan the cost of the main inorganic oxide. The ratio between the main inorganic oxide and the secondary inorganic oxide is selected so that no loss of property is observed compared with the main inorganic oxide alone. In particular, it has been observed that an amount of only 10% to 20% by weight of the main inorganic oxide can lead to the same advantages than 100% of the main inorganic oxide under the conversion process conditions. The combination of a main inorganic oxide with a secondary inorganic oxide thus allows to provide a less expensive catalyst without loss or significant loss of the benefits.

According to an embodiment, the secondary inorganic oxide has no benefits or a limited benefit compared to the main inorganic oxide. In this case, the secondary inorganic oxide is used as a carrier or a support for the main inorganic oxide.

According to another embodiment, the secondary inorganic oxide demonstrated some benefits in one or only some of the above-mentioned aspects of the conversion process, but limited or no benefits in the other aspects compared to the main inorganic oxide. For example, the secondary inorganic oxide allows to improve the regeneration process while the main inorganic oxide allows to improve several or all of the lifetime, the stability, the selectivity, the turn-over of the catalyst or the catalytic composition. The main and secondary inorganic oxides have thus complementary advantages, which globally provide more benefits.

According to an embodiment, the combination of the main inorganic oxide and the secondary inorganic oxide allows to improve at least one of the above-mentioned advantages compared to the use of only the main inorganic oxide. The combination of two inorganic oxides thus provides a synergistic beneficial effect.

The secondary inorganic oxide can be selected among cheapest compounds such as SiO₂, Al₂O₃, TiO₂, MgO, CaO and ZrO₂, or among more expensive compounds having demonstrated some benefits like Y₂O₃, La₂O₃ or Sm₂O₃ or other inorganic oxides. A mixture of secondary inorganic oxides can be envisaged in different ratios so as to better tune the advantages without losses or significant losses.

The main inorganic oxide is preferably selected among CeO₂, Y₂O₃, Sm₂O₃ or La₂O₃. More preferably CeO₂.

In the catalyst or catalytic composition according to the present invention, the ratio between the zeolite and the inorganic oxide is selected from 100 to 1 to 1 to 5 by weight. The ratio zeolite/inorganic oxide is thus 100/1, 10/1, 1/1, 1/2, 1/3, 1/4, 1/5, or any intermediate value.

According to an embodiment the zeolite is selected from ZSM-5, SSZ-13, BETA, ZSM-35, FAU, ZSM-22, BEA, USY and MOR, or a mixture thereof, and the main inorganic oxide is CeO₂, either alone or combined with a secondary inorganic oxide selected from MgO, ZrO₂, CaO, Y₂O₃, Sm₂O₃ or La₂O₃ or a mixture thereof.

According to an embodiment the inorganic oxide comprises CeO₂ as the main inorganic oxide and ZrO₂ or MgO as the secondary inorganic oxide in a ratio of 10/90 or 20/80 or 70/30 or 5/95 or 1/99 by weight.

The present invention further relates to a process for converting methanol and methanol derivatives into hydrocarbons, in particular low-molecular weight hydrocarbons, using a catalyst or a catalytic composition according to the present disclosure.

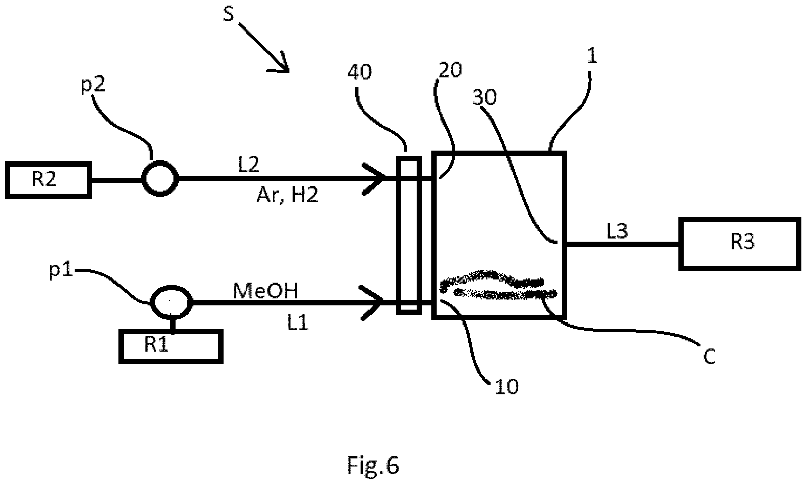

The conversion process comprises a step of filling a conversion chamber 1 with a suitable amount of a catalyst or catalytic composition **C** (figure 6). The catalyst or catalytic composition **C** is one of the above-described compositions or a mixture thereof in any suitable ratio. Depending on the needs, the suitable amount can be comprised between few grams and more than 1 kilogram of more than 10 kilogram of catalyst. The weighed catalyst **C** is arranged in the conversion chamber as solid material, either as a powder or as beads having a micrometre or millimetre diameter. It is preferably arranged on a support allowing a good contact between the feed gas and the catalyst **C.** For example, 1 kg of catalyst or catalytic composition **C** can be arranged for converting more than 400 kg or more than 500 kg or more than 600 kg or more than 10000 kg of methanol or methanol derivatives per conversion cycle. A conversion cycle defines here the process between two successive regenerations of the catalyst.

The process comprises a step of feeding the conversion chamber **1** with methanol or a methanol derivative. The feeding is operated through at least one inlet **10** at a suitable pressure. According to an embodiment, the feeding of methanol or methanol derivative is operated at atmospheric pressure. Alternatively, it occurs at a slight overpressure comprised between 1.1 and 5 bar, preferably between 1.2 and 3 or 4 bar, preferably lower than 2 bar. An inlet **10** here defines any suitable device adapted for feeding methanol. It can be for example a nozzle, an injector or any equivalent. The feeding of methanol can be made at a predetermined temperature such as room temperature or a temperature comprised between 50 and 900 K, such as 50 to 100 K or 150 to 300 K or higher than 350 K. To this end the process can comprise a step of warming the methanol at a suitable temperature prior to its feeding in the conversion chamber. Alternatively, the methanol or methanol derivative is fed at room temperature in a heated conversion chamber so that it is immediately or approximatively immediately heated at the suitable temperature during the process. Alternatively, a step of pre-heating the methanol or methanol derivatives at an intermediate temperature before its feeding in the conversion chamber **1** can be envisaged. Methanol or methanol derivatives can be fed to the conversion chamber through one or several first lines **L1** adapted to carry the methanol or methanol derivatives from a first tank **R1** to the conversion chamber **1.**

The process comprises a step of co-feeding the conversion chamber **1** with an inert gas such as argon or nitrogen. According to an embodiment, methanol or methanol derivatives and the inert gas are mixed in a proper ratio prior their feeding in the conversion chamber **1.** To this end, the necessary pumps and mixing units are provided. Other alternatives are possible.

The process comprises an optional step of co-feeding the methanol or methanol derivatives with a suitable flowrate of hydrogen.

The process comprises a step of contacting methanol or methanol derivatives fed in the chamber of conversion **1** with the catalyst or catalytic composition **C** to perform the conversion. The contact can be static, wherein the flow of methanol or methanol derivatives passes over the fixed bed of catalyst or catalytic composition **C.** The contact can be also accomplished by using a fluidized or moving bed of catalyst. Alternatively, a mechanical stirring can be implemented to improve the reaction. The conversion occurs inside the conversion chamber **1** at suitable pressure and temperature. Preferably the conversion occurs at a pressure lower than 5 or 3 or 2 bar. Depending on the needs, the conversion temperature is comprised between 50 and 900 K, preferably between 450 and 820 K.

The process comprises the step of collecting the hydrocarbon fractions resulting from the conversion out of the conversion chamber **1.** Collection of the hydrocarbon fractions can be made by mean of one or several outlets. Depending on the needs, one or several condensing steps, cooling steps, filtering steps can be implemented to collect the final fractions of hydrocarbons. According to an embodiment the collected hydrocarbon fractions represent alkenes having from 2 to 5 or from 3 to 5, or from 3 to 6 carbon atoms, in a selectivity of more than 70%, or more than 75% or more than 80%, up to more than 90% selectivity. The hydrocarbon fractions can be collected through one or several collection lines **L3** until one or several tanks **R3.** The hydrocarbon can be analysed online or offline to determine a conversion profile or a variation of the conversion profile.

According to an embodiment, the conversion process is a continuous process, meaning that the methanol or methanol derivatives are continuously fed to the conversion chamber **1** while the suitable hydrocarbon fractions are continuously collected. The temperature and the pressure inside the conversion chamber are controlled to perform the conversion.

During the process, some or all the parameters including conversion pressure, temperature pressure, methanol flowrate, inert gas flowrate, co-feeding gas flowrate, proportion of methanol and inert gas and co-feeding gas where applicable, can be predetermined according to the expected result. These parameters or some of them can be adapted according to the catalyst or the catalytic composition **C.** Alternatively, one or more of these parameters can be automatically adjusted according to predetermined algorithms based on corresponding values detected online by means of suitable sensors.

The process can comprise a step of regenerating the catalyst **C** after a predetermined cumulative turnover capacity of the catalyst or the catalytic composition has been reached. Alternatively, a regeneration step can be triggered upon detecting a degradation of the selectivity to the expected hydrocarbon fractions or a variation of the conversion profile. The regeneration step comprises feeding the conversion chamber **1** with oxygen at a suitable regeneration temperature. Depending on the used catalyst or catalytic composition **C,** the regeneration temperature can be comprised between 50 and 900 K, preferably between 670 and 850 K. The regeneration step defines the end of a conversion cycle and the start of a new conversion cycle. After a regeneration step, the steps of feeding methanol or methanol derivative, operating the conversion and collecting the hydrocarbon fraction, as described above can be iterated.

The process comprises a step of replacing the catalyst or catalytic composition **C** in the conversion chamber **1** after a partial or complete non-recoverable loss of activity. According to an embodiment, the catalyst or catalyst composition described here can be involved in more than 10 or more than 15 or more than 20 or more than 30 or more than 50 conversion cycles before being replaced.

The present invention further relates to a system **S** comprising a conversion unit and a catalyst or a catalytic composition **C** as described here (figure 6). The system **S** comprises a conversion unit adapted to convert methanol or methanol derivatives into hydrocarbons and an amount of suitable catalyst or catalytic composition **C** described here. The conversion unit comprises a conversion chamber **1** provided with at least one inlet **10, 20** adapted to feed the conversion chamber with the methanol or methanol derivatives under suitable conditions of temperature and pressure. The methanol or methanol derivative can be stored in a first tank or reservoir **R1** and supplied to the conversion chamber **1** by means of at least one first line **L1** and the necessary first pump **P1.** The conversion unit comprises at least one means to supply one or several gases such as inert gases or co-feeding gases. To this end, the conversion unit can comprise one or several gas tanks or reservoirs **R2** and corresponding gas lines or pipes **L2** adapted to supply the gases to the conversion chamber **1.** One or more adapted pumps **P2** can be implemented.

The conversion unit can comprise one or several intermediate units **40** upstream the conversion chamber **1,** adapted to prepare the feeding mixture comprising the methanol or methanol derivatives before its conversion. The intermediate unit or units **40** can be adapted to one or more of the following operations:
- Mixing methanol or methanol derivatives with one or several inert gases in suitable proportions such as MeOH/inert gas = 90/10 or 80/20 or 10/90 so as to provide a premix gas;
- Mixing a co-feeding gas, such as hydrogen or hydrocarbon, with the methanol or methanol derivative or with the premix gas in a suitable proportion;
- Pre-heating the premix gas and/or the methanol and/or the co-feeding gas at a suitable temperature;
- Compressing the premix gas and/or the methanol and/or the co-feeding gas at a suitable pressure;
- Controlling the flowrate of methanol and/or premix gas and/or co-feeding gas;

The conversion unit further comprises one or several collection lines L3 and corresponding outlet downstream the conversion chamber 1 so as to collect the converted fraction of hydrocarbon. The hydrocarbons can be stored in one or several dedicated tanks **R3.**

The conversion unit can comprise one or several sensors such as pressure sensors, temperature sensors, liquid or gas detection, analytical means such as hydrocarbon mass detection, as well as any suitable device.

The conversion unit can further comprise localized or remote piloting means comprising digital means, computation means, signal treatment means, data communication means, as well as any related device.

### Examples

The preparation of ZSM-5 catalyst was performed according to known procedures. For example, zeolite ZSM-5 with Si/AI of ca. 45 was prepared using TPAOH. Tetraethylorthosilicate and Al₂(SO₄)₃×18H₂O were used as silicon and aluminium sources, respectively. Synthesis gel composition was as follows: Al₂O₃:H₂SO₄:Na₂O:TPAOH:SiO₂:H₂O = 1:3:4.6:25:100:1250. The synthesis gel was aged at room temperature for 24 h, then transferred into a Teflon-lined stainless-steel autoclave, and hydrothermally crystallized at 443 K for 1 day under tumbling (30 rpm). The synthesized sample was calcined at 823 K for 5 h under dry air to remove the structure-directing agent. The calcined zeolite was ion-exchanged in a 0.1 M NH₄NO₃ aqueous solution three times and then calcined at 823 K for 5 h to obtain the protonated form.

Bulk MOZ catalyst was prepared by mixing 0.2 g of ZSM-5 catalyst with 0.2 g of ZSM-5 catalyst with Si/AI ratio of 40. The powders were weighted on a balance, transferred into a mixing mortar, and mixed with pestle for 2 minutes, with periodic removal of powder from the walls. Thereafter, the powders were transferred into a press die and pressed at 2 tons for 2 minutes. The catalyst was finally crushed and sieved to desired particle size fraction.

For the supported oxide, Ce(NO₃)₃×6H₂O (2.52 g) was dissolved in H₂O (2 g). The solution was dispersed over TiO₂ (10 g). The obtained solid was dried in vacuum (50 mbar) at 373 K for 12 h, and then calcined at 823 K for 5 h. The corresponding MOZ was obtained by following the identical protocol as described for bulk MOZ.

### Example 1a: Conversion vs cumulative turnover capacity

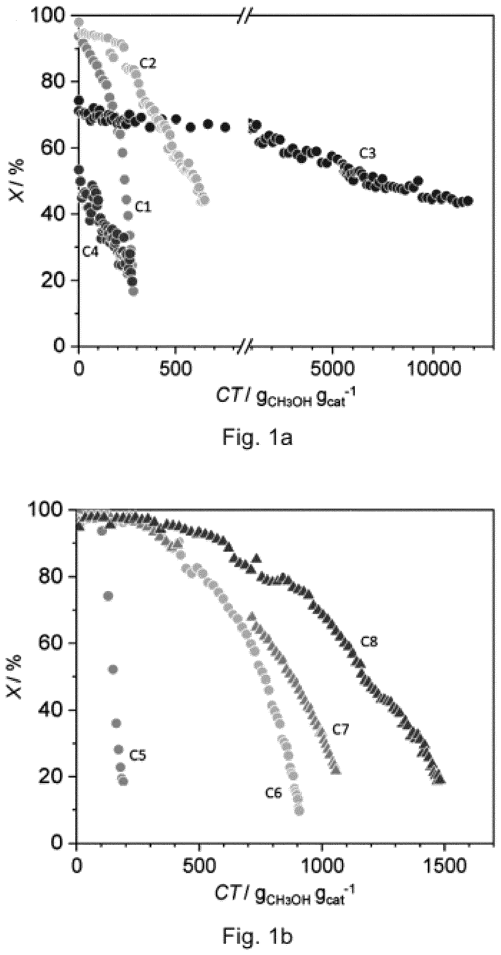

A catalyst based on zeolite ZSM-5(25), comprising a Si/AI ratio of 25 was mixed with CeO₂ or Y₂O₃ in different ratios to provide a mixed-oxide zeolite (MOZ). The conversion (X) determined in % vs cumulative turnover capacity (CT) determined in grams of methanol by gram of catalyst (gₘₑₜₕₐₙₒₗ g_{cat}⁻¹) was determined for the compositions of table 1a below. The corresponding values are shown in figure 1a.

The lifetime enhancement (LTE) was defined by the ratio between the cumulative turnover (CT) capacity of the mixed-oxide zeolite (MOZ) and the corresponding zeolite alone.

The following conditions were applied:
Flow rate of methanol per gram of catalyst WHSV = 44 gₘₑₜₕₐₙₒₗ g_{cat}⁻¹ h⁻¹,
Concentration of methanol in argon: CH₃OH:Ar = 11:89 mol%
Temperature T = 673 K
Pressure p = 1.5 bar

**Table 1a**

| Compositions | | LTE |
|---|---|---|
| C1 | ZSM-5(25) alone | |
| C2 | ZSM-5(25):CeO₂ 1:1 | ∼ 3.2 |
| C3 | ZSM-5(25):CeO₂ 1:5 | > 40 |
| C4 | ZSM-5(25):Y₂O₃ 1:5 | ∼ 1.5 |

Mixing ZSM-5 zeolite with CeO₂ increases the lifetime by about 3.2 to about 41 times. The beneficial effect of CeO₂ is significantly improved compared to Y₂O₃.

### Example 1b: Conversion vs cumulative turnover capacity under hydrogen co-feed

A catalyst based on Zeolite ZSM-5(40), comprising a Si/AI ratio of 40 was mixed with CeO₂ in a given ratio to provide a mixed-oxide zeolite (MOZ). The conversion (X) determined in % vs cumulative turnover capacity (CT) determined in grams of methanol by gram of catalyst (gₘₑₜₕₐₙₒₗ g_{cat}⁻¹) was determined under various concentrations of hydrogen (H₂) for the compositions of table 1b below. The corresponding values are shown in figure 1b.

The lifetime enhancement (LTE) was defined by the ratio between the cumulative turnover capacity (CT) of the mixed-oxide zeolite (MOZ) under hydrogen co-feed and the corresponding MOZ without hydrogen. The corresponding values are shown in figure 1b.

The following conditions were applied:
Flow rate of methanol per gram of catalyst WHSV = 76 gₘₑₜₕₐₙₒₗ g_{cat}⁻¹ h⁻¹,
Concentration of methanol in argon co-feed with hydrogen: CH₃OH:Ar:H₂ = 20:(80/60/0):(0/20/80) mol%
Temperature T = 773 K
Pressure p = 1.6 bar

**Table 1b**

| compositions | | H₂ | LT |
|---|---|---|---|
| C5 | ZSM-5(40) | 0% | |
| C6 | ZSM-5(40):CeO₂ 1:1 | 0% | ∼ 4 |
| C7 | ZSM-5(40):CeO₂ 1:1 | 20% | ∼ 5.1 |
| C8 | ZSM-5(40):CeO₂ 1:1 | 100% | ∼ 7 |

The addition of hydrogen co-feed over MOZ catalysts increases the lifetime of the catalyst, already at a pressure below 2 bar.

### Example 2: regeneration of the catalyst

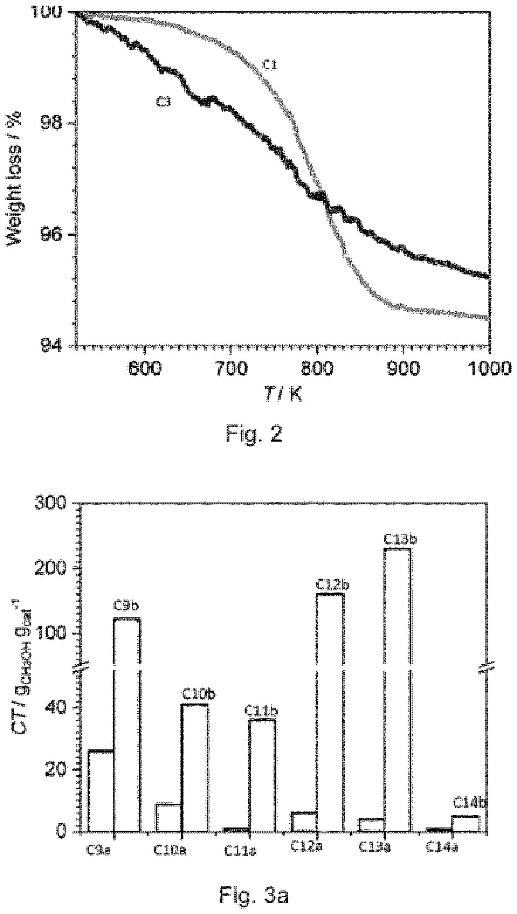

Catalysts deactivated through methanol-to-hydrocarbons conversion under standard conditions were subjected to a regeneration process under high temperatures. The oxidative regeneration of a catalyst based on ZSM-5(25):CeO₂ 1:5 (composition C3) was followed by thermogravimetry and compared with the corresponding zeolite alone (Composition C1). The corresponding results are provided in figure 2.

The oxidation of coke in the MOZ catalyst occurs at significantly lower temperature in comparison with the corresponding zeolite alone, at temperatures above 530 K.

### Example 3a: Lifetime extension of different Zeolites with CeO₂

Catalysts based on various zeolites were involved in a methanol-to-hydrocarbons conversion in absence of CeO₂ and as MOZ when mixed with CeOz in a ratio of zeolite:CeO₂ of 1:1. The conversion (X) determined in % vs cumulative turnover capacity (CT) determined in grams of methanol by gram of catalyst (gₘₑₜₕₐₙₒₗ g_{cat}⁻¹) was determined for the compositions of table 3a below. The corresponding values are shown in figure 3a.

The lifetime enhancement (LTE) was defined by the ratio between the cumulative turnover capacity (CT) of the mixed-oxide zeolite (MOZ) and the corresponding zeolite alone.

The following conditions were applied:
Flow rate of methanol per gram of catalyst
WHSV = 4.8 gₘₑₜₕₐₙₒₗ g_{cat}⁻¹ h⁻¹, (C10a, C10b, C11a, C11b),
WHSV = 9.6 gₘₑₜₕₐₙₒₗ g_{cat}⁻¹ h⁻¹ (C12a, C12b, C13a, C13b, C14a, C14b),
WHSV = 19 gₘₑₜₕₐₙₒₗ g_{cat}⁻¹ h⁻¹ (C9a, C9b).

Rate of methanol in argon:
CH₃OH:Ar = 11:89 (C10a, C10b, C11a, C11b, C12a, C12b, C13a, C13b, C14a, C14b) or
CH₃OH:Ar = 5.6:94.4 (C9a, C9b) mol%

### Temperature

T = 773 K (C13a, C13b),
T = 723 K (C12a, C12b),
T = 673 K (C14a, C14b, C12a, C12b, C11a, C11b, C10a, C10b, C9a, C9b)
Pressure p = 1.6 bar

**Table 3a**

| compositions | | LTE |
|---|---|---|
| C9a | BEA(19) | ∼ 4.7 |
| C9b | BEA(19):CeO₂ 1:1 | |
| C10a | SSZ-13(10) | ∼ 4.7 |
| C10b | SSZ-13(10):CeO₂ 1:1 | |
| C11a | ZSM-35(10) | ∼ 36 |
| C11b | ZSM-35(10):CeO₂ 1:1 | |
| C12a | USY(40) | ∼ 26 |
| C12b | USY (40):CeO₂ 1:1 | |
| C13a | ZSM-22(40) | ∼ 56 |
| C13b | ZSM-22(40):CeO₂ 1:1 | |
| C14a | MOR(15) | ∼ 8 |
| C14b | MOR(15):CeO₂ 1:1 | |

The lifetime of unstable zeolites is significantly increased when mixed with CeO₂.

### Example 3b: selectivity of C₃-C₅ alkenes for ZSM-35 and ZSM-5 based catalysts

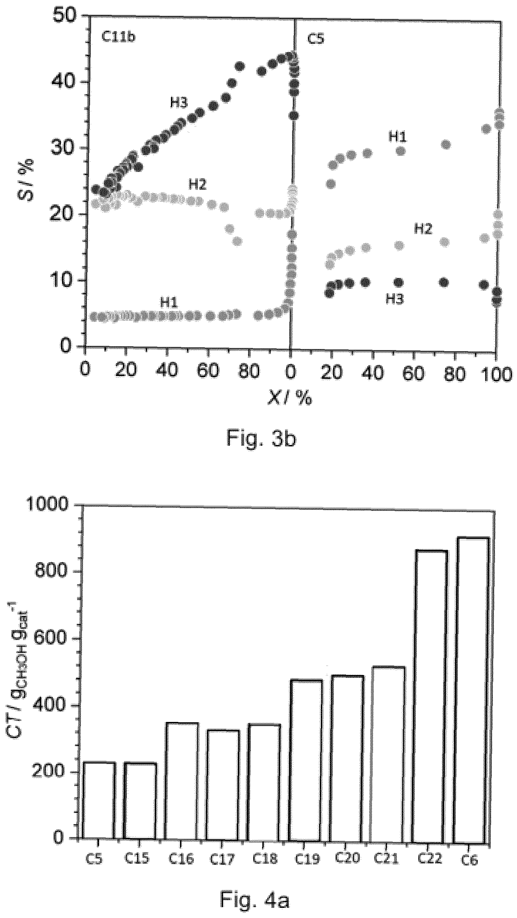

Catalysts ZSM-35(10):CeO₂ 1:1 (composition C11b) and ZSM-5 (40) alone (composition C5) were involved in a methanol-to-hydrocarbons conversion process under the same conditions. The conversion (S), determined in %, in C₃H₆ (H1) C₄H₈ (H2) and C₅H₁₀ (H3) hydrocarbons was followed for the two catalysts. The corresponding results are shown in figure 3b.

### Example 4a: Lifetime enhancement of bulk oxide zeolites

The maximum cumulative turnover capacity of catalysts based on ZSM-5(40) mixed with various inorganic oxides as a bulk mixture in a ratio ZSM-5(40):oxide of 1:1, except for CaO wherein the ratio is 1:0.1, was determined in the conditions of methanol-to-hydrocarbons conversion.

The life-time enhancement (LTE) was defined by the ratio between the cumulative turnover capacity (CT) of the mixed-oxide zeolite (MOZ) and the corresponding zeolite alone. The corresponding results are provided in the table 4a below and in figure 4a:

The following conditions were applied:
Flow rate of methanol per gram of catalyst WHSV = 76 gₘₑₜₕₐₙₒₗ g_{cat}⁻¹ h⁻¹,
Rate of methanol in argon: CH₃OH:Ar = 20:80 mol%
Temperature T = 773 K
Pressure p = 1.6 bar

**Table 4a**

| compositions | | LTE |
|---|---|---|
| C5 | ZSM-5(40) alone | |
| C15 | ZSM-5(40):TiO₂ 1:1 | ∼ 1 |
| C16 | ZSM-5(40):CaO 1:0.1 | ∼ 1.5 |
| C17 | ZSM-5(40):Al₂O₃ 1:1 | ∼ 1.4 |
| C18 | ZSM-5(40):ZrO₂ 1:1 | ∼ 1.5 |
| C19 | ZSM-5(40):La₂O₃ 1:1 | ∼ 2.1 |
| C20 | ZSM-5(40):Sm₂O₃ 1:1 | ∼ 2.2 |
| C21 | ZSM-5(40):MgO 1:1 | ∼ 2.3 |
| C22 | ZSM-5(40):Y₂O₃ 1:1 | ∼ 3.8 |
| C6 | ZSM-5(40) : CeO₂ 1:1 | ∼ 4 |

Although the best lifetime enhancement is obtained with CeO₂, several other oxides have demonstrated a significant lifetime enhancement.

### Example 4b: Lifetime enhancement of zeolites with supported oxides

The maximum cumulative turnover capacity of catalysts based on ZSM-5(40) combined with CeO₂ supported by various carriers was determined in the conditions of methanol-to-hydrocarbons conversion. The ratio ZSM-5 (40):oxide was 1:1 considering the total amount of oxides. The amount of CeO₂ in the oxides was 10 wt%.

The conditions were the same as those of example 4a.

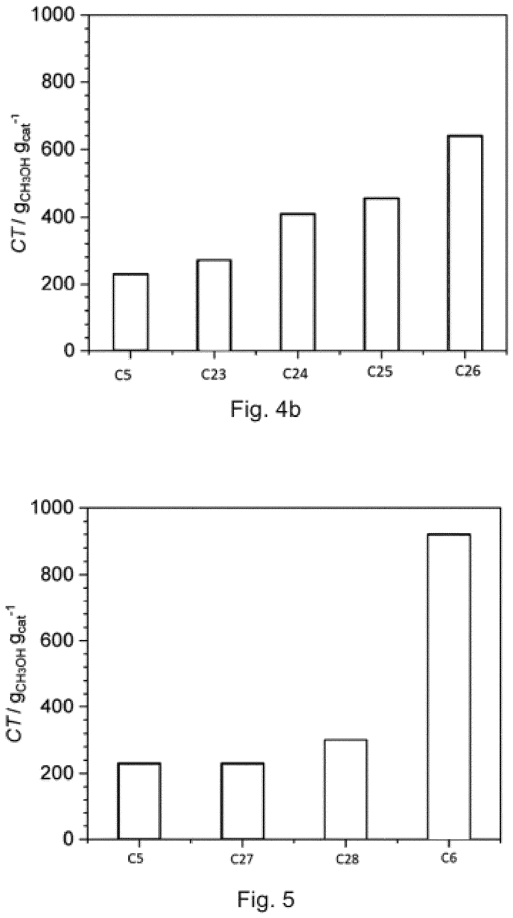

The lifetime enhancement (LTE) was defined by the ratio between the cumulative turnover capacity (CT) of the zeolite combined with supported CeO₂ and the corresponding zeolite alone. The corresponding results are provided in the table 4b below and in figure 4b:

**Table 4b**

| compositions | | LTE |
|---|---|---|
| C5 | ZSM-5(40) alone | |
| C23 | ZSM-5(40):CeO₂/SiO₂ 1:1 | ∼ 1.2 |
| C24 | ZSM-5(40):CeO₂/Al₂O₃ 1:1 | ∼ 1.8 |
| C25 | ZSM-5(40):CeO₂/TiO₂ 1:1 | ∼ 2 |
| C26 | ZSM-5(40):CeO₂/ZrO₂ 1:1 | ∼ 2.8 |

The lifetime enhancement is still substantial even at reduced amount of supported main oxide.

### Example 5: comparative lifetime enhancement of zeolites with supported oxides

The maximum cumulative turnover capacity of catalysts was determined in the conditions of methanol-to-hydrocarbons conversion. Examples C27 and C28 represent the catalysts prepared by supporting CeO₂ over ZSM-5 at 2 different loadings of 1 and 10 wt%.

The lifetime enhancement (LTE) was defined by the ratio between the cumulative turnover capacity (CT) of the catalysts prepared by supporting CeO₂ over ZSM-5 at 2 different loadings of 1 and 10 wt%. The corresponding results are provided in the table 5 below and in figure 5.

The following conditions were applied:
Flow rate of methanol per gram of catalyst WHSV = 76 gₘₑₜₕₐₙₒₗ g_{cat}⁻¹ h⁻¹,
Concentration of methanol in argon: CH₃OH:Ar = 20:80 mol%
Temperature T = 773 K
Pressure p = 1.6 bar

| compositions | | LTE |
|---|---|---|
| C5 | ZSM-5(40) alone | |
| C27 | ZSM-5(40) + CeO₂ 1 wt% | ∼ 1 |
| C28 | ZSM-5(40) + CeO₂ 10wt% | ∼ 1.3 |
| C6 | ZSM-5(40):CeO₂ of 1:1 | ∼ 4 |

The preparation method based on mixing leads to better performance than conventional supporting/impregnation of zeolite with CeO₂.

## Claims

1. A porous solid catalyst or catalytic composition (C) adapted for the conversion of methanol and/or methanol derivatives into low-molecular weight hydrocarbons having from 2 to 15 carbon atoms, comprising:
- one or more zeolites selected from ZSM-57, ZSM-58, Sigma-1, ITQ-3, ITQ-13, ITQ-33, SUZ-4, MCM-22, EU-1, UZM-9, UZM-12, SAT-7, ZK-5, RHO, RUB-13, UZM-5, SSZ-16, SSZ-24, SSZ-39, SSZ-52, SSZ-98, ZSM-5, ZSM-11, SSZ-13, BETA, ZSM-35, FAU, ZSM-22, BEA, USY and MOR, or a mixture thereof,
- at least one inorganic oxide selected from mono-, di-, tri-, and tetra-oxidated alkali metals, alkaline rare earth metals, rare earth metals and transition metals such as Sm₂O₃, Y₂O₃, La₂O₃, MgO, CaO, SrO, BaO, ZrOz, CeOz, Eu₂O₃, Pr₂O₃, Nd₂O₃,Pm₂O₃, Tb₂O₃, Dy₂O₃,Ho₂O₃, Er₂O₃, SnO₂, ZnO, Mo₂O₃, SiO₂, Al₂O₃, TiO₂, or a mixture thereof, or their doped forms, wherein dopant is one or more alkaline, alkaline earth, transition, or rare-earth metals,
**characterized in that** the ratio of zeolite:inorganic oxide is comprised between 100:1 and 1:50, preferably between 1:1 and 1:5.

2. Porous catalyst or catalyst composition according to claim 1, wherein said inorganic oxide comprises CeO₂, either alone or combined with one or more of SiO₂, Al₂O₃, TiO₂, Sm₂O₃, Y₂O₃, La₂O₃, MgO, CaO, ZrO₂, Mo₂O₃.

3. Porous solid catalyst or catalytic composition according to claim 1, wherein said zeolite or mixture thereof and said inorganic oxide or mixture thereof are mixed in the bulk to provide mixed-oxide zeolite (MOZ).

4. Porous solid catalyst or catalytic composition according to claim 1, wherein said oxide or mixture thereof is combined to said zeolite or mixture thereof as a supported inorganic oxide, and it comprises a main inorganic oxide and a secondary inorganic oxide in a ratio of main inorganic oxide/secondary inorganic oxide comprised between 1 / 99 and 49/51, wherein the secondary inorganic oxide is selected among SiO₂, Al₂O₃, TiO₂, MgO, ZrOz, CaO, or a mixture thereof and wherein the main inorganic oxide is selected among CeO₂, Y₂O₃, Sm₂O₃ or La₂O₃, preferably CeO₂.

5. Porous solid catalyst or catalytic composition according to claim 4, wherein said supported inorganic oxide comprises 1 wt%, 10 wt% or 20 wt%, or 50 wt% or 80 wt% of CeO₂ with a carrier, wherein the carrier is selected from SiO₂, Al₂O₃, TiO₂, ZrO₂, Nb₂O₅, Ta₂O₅, mesoporous and microporous silicates and aluminosilicates or mixture thereof.

6. Porous solid catalyst or catalytic composition according to one of claims 1 to 5, said zeolite being selected from ZSM-5(x), BEA(x), SSZ-13(x), ZSM-35(x), USY(x), ZSM-22(x) and MOR(x), x defining the Si/AI ratio and being equal to 1, 10, 15, 19, 25, 40, 80, 200, or 1000.

7. Porous solid catalyst or catalytic composition according to claims 1 to 6, said zeolite being selected among ZSM-5(25), ZSM-5(40), BEA(19), SSZ-13(10), SSZ-13(10), ZSM-35(10), USY(40), ZSM-22(40) and MOR(15).

8. Porous solid catalyst or catalytic composition according to claims 1 to 7, wherein said catalyst or catalytic composition is one of the following: .
| Compositions | |
|---|---|
| C2 | ZSM-5(25):CeO₂ 1:1 |
| C3 | ZSM-5(25):CeO₂ 1:5 |
| | |
|---|---|
| C4 | ZSM-5(25):Y₂O₃ 1:5 |
| C6 | ZSM-5(40):CeO₂ 1:1 |
| C7 | ZSM-5(40):CeO₂ 1:1 |
| C8 | ZSM-5(40):CeO₂ 1:1 |
| C9b | BEA(19):CeO₂ 1:1 |
| C10b | SSZ-13(10):CeO₂ 1:1 |
| C11b | ZSM-35(10):CeO₂ 1:1 |
| C12b | USY (40):CeO₂ 1: 1 |
| C13b | ZSM-22(40):CeO₂ 1:1 |
| C14b | MOR(15):CeO₂ 1:1 |
| C15 | ZSM-5(40):TiO₂ 1:1 |
| C16 | ZSM-5(40):CaO 1:0.1 |
| C17 | ZSM-5(40):Al₂O₃ 1:1 |
| C18 | ZSM-5(40):ZrO₂ 1:1 |
| C19 | ZSM-5(40):La₂O₃ 1:1 |
| | |
|---|---|
| C20 | ZSM-5(40):Sm₂O₃ 1:1 |
| C21 | ZSM-5(40):MgO 1:1 |
| C22 | ZSM-5(40):Y₂O₃ 1:1 |
| C23 | ZSM-5(40): 10%CeO₂/SiO₂ 1: 1 |
| C24 | ZSM-5(40): 10%CeO₂/Al₂O₃ 1:1 |
| C25 | ZSM-5(40): 10%CeO₂/TiO₂ 1:1 |
| C26 | ZSM-5(40): 10%CeO₂/ZrO₂ 1: 1 |
| C27 | ZSM-5(40) + CeO₂ 1 wt% |
| C28 | ZSM-5(40) + CeO₂ 10 wt% |

9. A process of conversion of methanol or methanol derivatives into low-molecular weight hydrocarbons comprising the steps :
- (E1) of contacting an amount of methanol or methanol derivative (Q_{MeOH}) with a suitable amount of catalyst or catalytic composition (Qc) according to one of claims 1 to 8 at a conversion temperature (Tcv) and at a conversion pressure (pcv) so as to convert said methanol or methanol derivatives into hydrocarbons, such as alkenes, having from 2 to 12, preferably 2 to 5, carbon atoms during a conversion cycle,
- (E2) of regenerating said suitable amount of catalyst or catalytic composition (Qc) at the end of said conversion cycle, at a regeneration temperature (Tr) and a regeneration pressure (Pr), and
- Iterating steps (E1) and (E2) at least 10, 15, 20, 25 or 30 times.

10. Process according to claim 9, wherein said methanol or methanol derivative is combined with an inert gas such as argon or nitrogen in a weight ratio of methanol/gas of 10/90, 20/80, 70/30, or 99/1.

11. Process according to one of claims 9 and 10, wherein said methanol or methanol derivative is co-fed with hydrogen (H₂) and or another co-feed such as water (steam) or hydrocarbon.

12. Process according to one of claims 9 to 11, wherein said amount of methanol or methanol derivatives (Q_{MeOH}) and said suitable amount of catalyst or catalytic composition (Qc) is defined so that the weight ratio Q_{MeOH}/Qc is higher than 400/1, or 500/1, or 600/1, or 1000/1 for a conversion cycle.

13. Process according to one of claims 9 to 12, wherein said conversion temperature (Tcv) is lower than 850 K, or lower than 600 K or lower than 400 K wherein the conversion pressure (pcv) is lower than 30, 10, 5 or 4 or 3 or 2 bar.

14. Process according to one of claims 9 to 13, wherein said regeneration temperature (Tr) is comprised between 50 and 850 K and said regeneration pressure (pr) is lower than 30 bar.

15. System (S) adapted to convert methanol or methanol derivatives into low-molecular weight hydrocarbons comprising:
- A conversion unit, and
- A suitable amount (Qc) of a catalyst or catalytic composition (C) according to one of claims 1 to 8.
